# EUROPEAN PATENT SPECIFICATION

(11) **EP 2 924 021 B1**
(45) Date of publication and mention of the grant of the patent: **28.08.2019**
(21) Application number: 13857626.9
(22) Date of filing: 13.12.2013
(51) Int. Cl.: C07C 319/20, C07C 323/62, A61K 31/167, A61P 31/18, C07C 317/48, C07C 317/50, C07C 323/63, C07C 237/44, C07D 295/155, C07F 9/40

(54) **ANTI-HIV COMPOUND AND PREPARATION METHOD AND USE THEREOF**
ANTI-HIV-VERBINDUNG SOWIE HERSTELLUNGSVERFAHREN UND VERWENDUNG DAVON
COMPOSÉ ANTI-VIH ET PROCÉDÉ DE PRÉPARATION ET UTILISATION CORRESPONDANTS

(30) Priority: 26.11.2012 CN 201210484783
(43) Date of publication of application: 30.09.2015
(73) Proprietor: Sichuan University, Sichuan 610065 (CN)
(72) Inventor: LI, Rui, Chengdu Sichuan 610065 (CN); WEI, Yuquan, Chengdu Sichuan 610065 (CN)
(74) Representative: MacLachlan & Donaldson
(86) International application number: PCT/CN2013/089341
(87) International publication number: WO 2014/079398

(56) References cited:
- WO-A1-2012/097550
- WO-A2-2007/044565
- WO-A2-2010/123591
- CN-A- 103 183 625
- ROBIN NATHANS ET AL: "Small-molecule inhibition of HIV-1 Vif", NATURE BIOTECHNOLOGY, vol. 26, no. 10, 21 October 2008 (2008-10-21), pages 1187-1192, XP055074677, ISSN: 1087-0156, DOI: 10.1038/nbt.1496
- NIELSEN, F.E. ET AL.: '2-(4-Methoxyphenoxy)-5-nitro-N-(4-sulfamoy lphenyl) benzamide activates Kir6.2/SURl KATP channels' BIOORGANIC & MEDICINAL CHEMISTRY LETTERS vol. 14, no. 23, October 2004, pages 5727 - 5730, XP004611108

## Description

### FIELD OF THE INVENTION

The present invention relates to the technical field of chemically synthesized drugs, in particular to an anti-HIV drug, a preparation method and uses thereof.

### BACKGROUND OF THE INVENTION

Acquired immune deficiency syndrome (AIDS), which disseminates quickly in the world, has become significant public health event and social hot spot.

AIDS was identified as a disease in 1981 by Centers for Disease Control and Prevention of USA. China experienced its first case of AIDS in 1985. In the past two decades, more and more cases were reported. Nowadays, it has already been spread in many areas of the world, Since China is a country of vast territory and large population, together with the rencently frequent international contacts, the number of HIV infections increased year by year..AIDS is a spectrum of conditions caused by the human immunodeficiency virus (HIV), with poor prognosis and high mortality rate. According to the related report of UNAIDS, the population of HIV infections around the world is more than 39 million. Nowadays, India takes over South Africa as the largest population of HIV-infected individuals in the world.

Thus, finding a way to stop the spread of AIDS has become the focus of the world. Most of the Anti-AIDS drugs in clinic are HIV-1 reverse transcriptase inhibitors and HIV-1 protease inhibitors at present time. In humans, the apolipoprotein B mRNA-editing enzyme catalytic polypeptidelike 3G (APOBEC3G, A3G) is a major host-cell factor which can severely weaken the infectivity of HIV-1. It was proved that HIV-1 viral infectivity factor (Vif) can protect the virus from A3G-mediated viral cDNA hypermutation. Therefore, to protect A3G from degradation, it is very crucial to design inhibitors targeting Vif. In addition, development of multi-target inhibitor of Vif accompanied by other enzymes may improve the therapeutic effect and reduce the virus resistance.

The inventor of this invention endeavored to find the new HIV inhibitors. The inventor has synthesized a series of N-phenyl-2-thiophenylbenzamide derivatives and N-phenyl-2- thiol benza-mide derivatives as following:

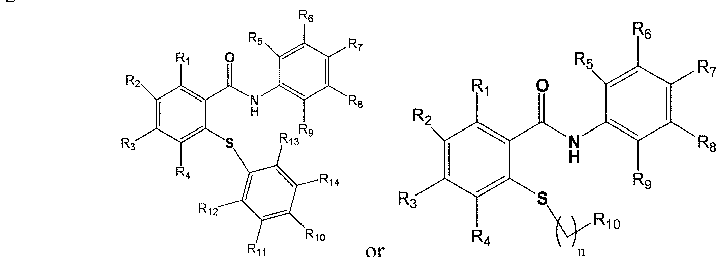

However, those compounds shows general activity, high cytotoxicity, poor solubility and low bioavailability which restrict its application.

In the follow-up project, the inventor found that R₁ or R₂ substituted by -NH₂ or -NH₂-R can obviously improve the anti-HIV activity, solubility, druggability and reduce drug toxicity.

WO 2007/044565 A2 discloses compounds and compositions for inhibiting Vif and methods for treating viral infection, e.g., HIV infection. The Vif inhibitor compound has Formula I:

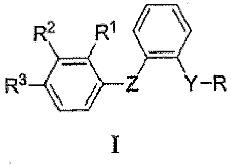

ROBIN NATHANS ET AL: "Small-molecule inhibition of HIV-1 Vif",NATURE BIOTECHNOLOGY, vol. 26, no. 10, (2008), pages 1187-1192 discloses a small molecule, RN-18, that antagonizes Vif function and inhibits HIV-1 replication only in the presence of A3G. RN-18 increases cellular A3G levels in a Vif-dependent manner and increases A3G incorporation into virions without inhibiting general proteasome-mediated protein degradation. RN-18 enhances Vif degradation only in the presence of A3G, reduces viral infectivity by increasing A3G incorporation into virions and enhances cytidine deamination of the viral genome. These results demonstrate that the HIV-1 Vif-A3G axis is a valid target for developing small molecule based new therapies for HIV infection or for enhancing innate immunity against viruses.

WO 2012/097550 A1 discloses Cyclohydrocarbyl formamide derivatives of general formula (I), preparation methods and uses thereof are provided. The cyclohydrocarbyl formamide derivatives of general formula (I) target on the auxiliary protein Vif and protect the natural anti-virus capability of APOBEC3G by inhibiting the function of Vif. And, new choices for the anti-HIV, anti-HCV or anti-HBV treatments are provided.

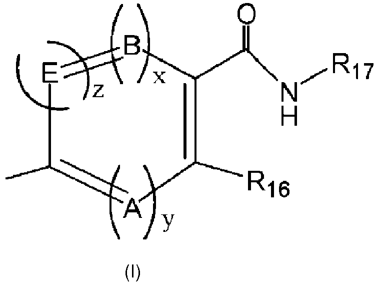

### SUMMARY OF THE INVENTION

One embodiment of the present invention is directed to a new HIV inhibitor of formula (I) or its pharmaceutically acceptable salt.

In one embodiment, the HIV inhibitors of the present invention have the following structural formula (III-11-111): 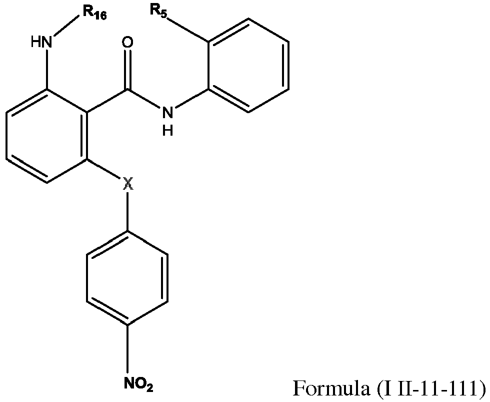 wherein:
R₅ is a straight or branched C₁₋₈ alkoxy group;
X is S, O or SO₂;
R₁₆ is H
or an anti-HIV compound as represented by a structure selected from the following group:

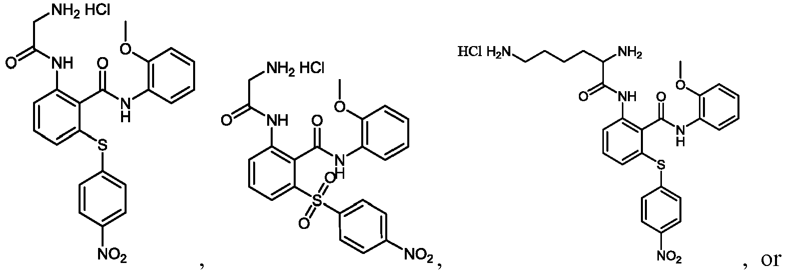

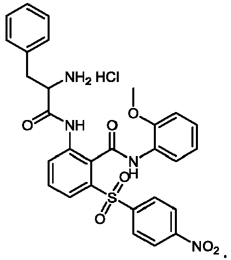

The term "alkoxy" as used herein refers to a straight or branched alkyl group attached to oxygen (alkyl-O-). "C1-8 alkoxy" refers to an alkoxy group which has 1-8 carbon atom(s). Exemplary alkoxy groups include, but are not limited to methoxy, ethoxy, isopropoxy. Particularly R₅ is C₁₋₄ alkoxy, further particularly C₁₋₂ alkoxy, and most particularly R₅ is methoxyl.

The ideal way of administration for AIDS drugs is oral. Generally, oral bioavailability has close relationship with the solubility. In this invention, although the derivative 46 with R₁₆=H shows the strongest antiviral activity, it bears the general solubility. In order to improve the oral bioavailability, the inventor used those amino acids to react with the amine group and acquired the corresponding prodrugs. Those prodrugs can be hydrolyzed to form the active compounds in the body. For example, compound 67 can be hydrolyzed to generate the active compound 46.

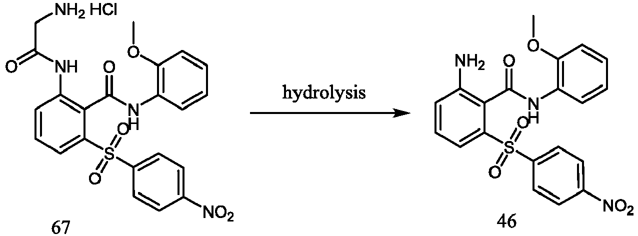

The present invention especially relates to the compound of formula (III-11-111), structural format is as follows: 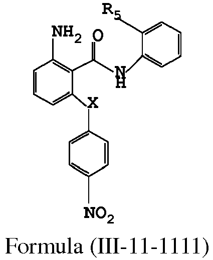 Wherein:
when R₅ is C₁₋₈ alkoxy, the term "alkoxy" as used herein refers to a straight or branched alkyl group attached to oxygen (alkyl-O-), and particularly R₅= methoxyl; X=S, SO₂ or O; (compound 25, 46,48)

### Examples of formula compounds are shown below.

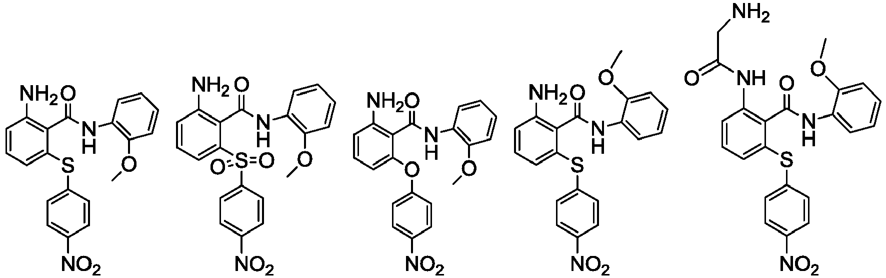

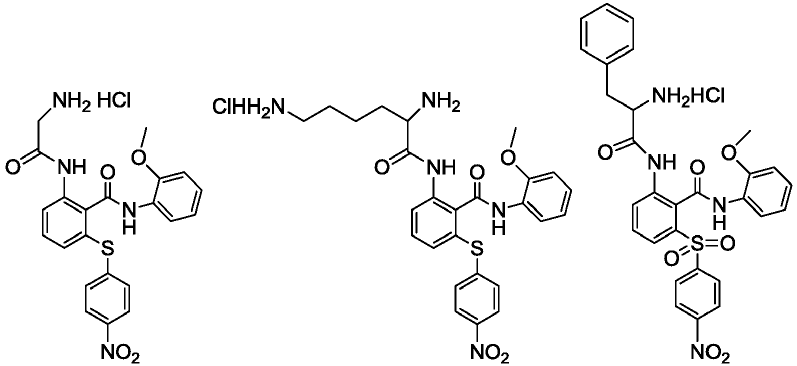

The present invention also provides for herein unclaimed processes of making the pharmaceutically acceptable salts. The term "salts" as used herein refers to hydrochloride, sulfate, phosphate or nitrate and particularly hydrochloride.

The present invention also provides compound's application for use as an anti-HIV drug, antineoplastic drug or anti-hepatitis drug.

Moreover, the present invention also provides for compound's application as HIV-1 or HIV-2 inhibitor.

Formula I compounds of the present invention are prepared using the method described below: 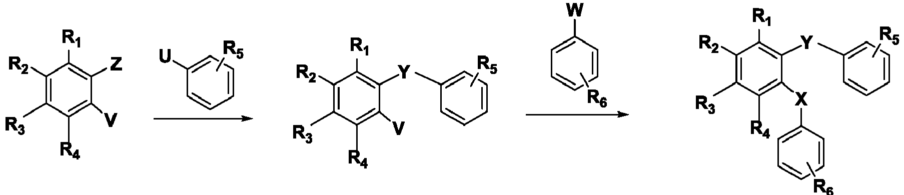
Wherein X=S, SO₂ or O;
Y=CONR₇;
Z=COOH or COCl;
U=NR₇H;
V=Cl, Br or I; W=SH or OH.
When X=S, the synthesis route are described below: 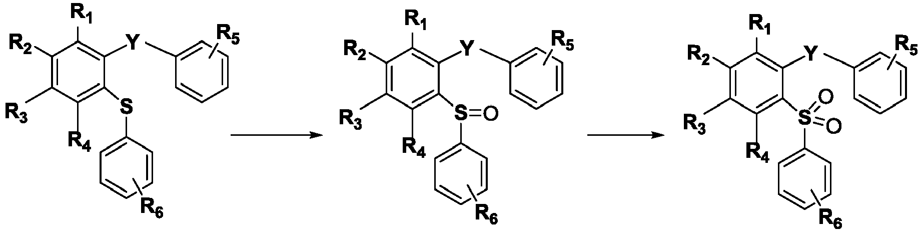
Wherein Y=CONR₇

### The beneficial effect of the invention:

The compounds have anti-HIV-1 and anti-HIV-2 virus activity, and have a C8166 therapeutic index as high as 2081.59 and an H9 therapeutic index as high as 303.03. Furthermore, the compounds have high solubility (1290-2845.5 µg/ml in an aqueous solution), and can be formulated into an oral formulation.

### BRIEF DESCRIPTION OF THE DRAWINGS

The following detailed description, given by way of example, but not intended to limit the invention solely to the specific embodiments described, may best be understood in conjunction with the accompanying drawings, in which:
FIG.1 shows a plasma concentration image of compound 67 at 100mg/Kg by gavage.
FIG.2 shows a plasma concentration image of compound 67 at 50mg/Kg by intravenous.
FIG.3 shows a degradation image of compound 67 in weak acid solution.
FIG.4 shows a degradation image of compound 67 under mild alkaline conditions.

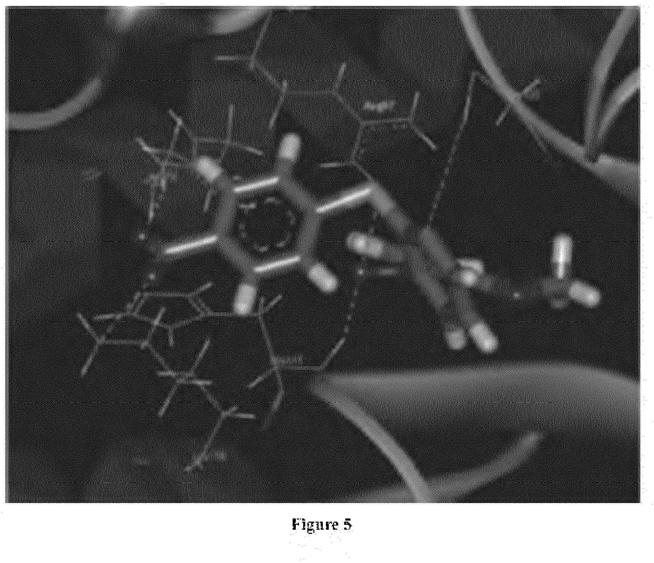

FIG.5 shows a possible binding mode between Vif and compound 46.

### EXPERIMENTAL EXAMPLES

### Example 1

### General Procedure for 2-amino-N-(2-methoxyphenyl)-6-(4-nitrophenylthi-o)benzamide (compound 25)

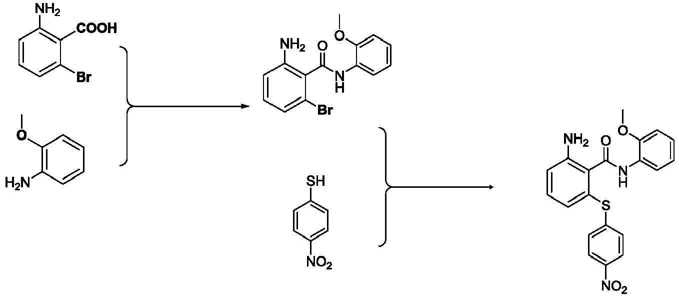

### Synthesis of 2-amino-6-bromo-N-(2-methoxyphenyl)benzamide

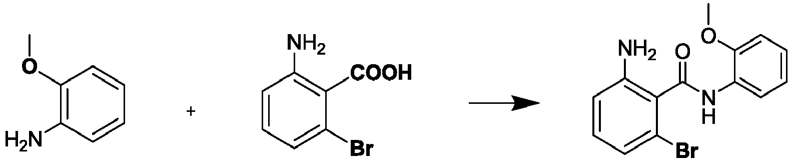

A mixture of 2-methoxyaniline (1.0 equiv.), 2-amino-6-bromobenzoic acid (1.0 equiv.), EDCI(1.2 equiv.) in THF (10ml) was stirred at room temperature for 5 hrs. After the reaction was completed, the THF was removed by vaccum distillation. The resulting reaction mixture was extracted with EtOAc (3x20 mL). The combined oiganic extracts were washed with water (20 mL), brine (20 mL), dried over MgSO₄ and concentrated in vacuo. The residue was purified over silica gel using EtOAc:Hexanes (1:4) as the eluent to afford intermediate **25** (yield 85%) as a brown crystalline solid.
¹H NMR (400 MHz, DMSO) δ = 3.80 (s, 3H), 5.33 (s, 2H), 6.72 (d, J = 8Hz, 1H), 6.78 (d, J = 8Hz, 1H), 6.99 (m, 2H), 6.07 (d, J = 8.4Hz, 1H), 7.18 (t, J = 7.6Hz, 1H), 7.85 (t, J = 7.2Hz, 1H), 9.43 (s, 1H).
ESI-MS: [M+Na]⁺m/z 346.

### Synthesis of 2-amino-N-(2-methoxyphenyl)-6-(4-nitrophenylthi-o)benzamide

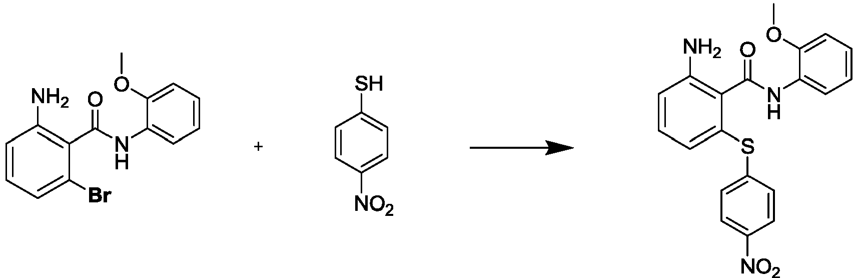

A mixture of 2-amino-6-bromo-N-(2-methoxyphenyl)benzamide (1.0 equiv.), 4-nitrobenzenethiol (2.0 equiv.), nanometer copper powder (0.5 equiv.) and anhydrous potassium carbonate (3.0 equiv.) were mixed in DMF (40 mL) at RT. Then the reaction flask was heated in an oil bath at 65 °C. After 8h, the heating bath was removed and the reaction flask was allowed to cool to room temperature. The reaction mixture was filtered and the liquid layer was diluted with water (40 mL), extracted with ethyl acetate (3 × 20 mL), washed with water (6 × 15 mL), dried over anhydrous Na₂SO₄, filtered, and concentrated under reduced pressure. The residue was purified by silica gel column chromatography (1:3 v/v ethyl acetate/petroleum ether) to provide the product compound 6 as a yellow amorphous solid (yield 75%).
¹H NMR (400 MHz, CDCl₃) δ = 3.69 (s, 3H), 4.54 (br, 2H), 6.84 (d, J = 8Hz, 2H), 6.95 (m, 2H), 7.06 (t, J = 8Hz, 1H), 7.22 (m, 3H), 8.03 (d, J = 7.6Hz, 2H), 8.23 (s, 1H), 8.32 (d, J = 7.6Hz, 1H).
ESI-MS: [M+H]⁺ m/z 396.

### Comparative Example 1

### General Procedure for N-(2-methoxyphenyl)-2-((4-nitrophenyl)thio)-5-(trifluoromethyl)benzamide (compound 22)

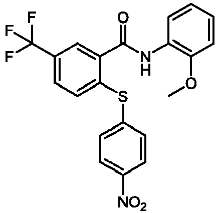

Prepared by proceeding in similar manner to example 1, staring from N-(2-methoxyphenyl)-2-((4-nitrophenyl)thio)-5-(trifluoromethyl)benzamide. Yellow solid; 77.9% yield;
¹H NMR (400 MHz, CDCl₃) δ = 3.85 (s, 3H), 6.90 (d, J = 8.1Hz, 1H), 6.99 (t, J = 7.6Hz, 1H), 7.11 (td, J = 8.0, 1.5, 1H), 7.44 (d, J = 8.8, 2H), 7.52 (d, J = 8.3Hz, 1H), 7.67 (dd, J = 8.3, 1.5, 1H), 7.97 (s, 1H), 8.14 (t, J = 5.7, 2H), 8.39 (d, J = 6.2, 2H).
ESI-MS: [M+H]⁺ m/z 449.

### Comparative Example 2

### General Procedure for N-(2-methoxyphenyl)-5-nitro-2-(4-nitrophenylthio)benzamide (compound 23)

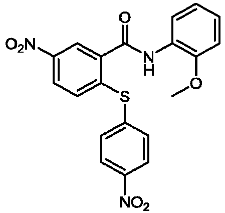

Prepared by proceeding in similar manner to example 1, staring from 2-bromo-5-nitrobenzoic acid. Yellow solid; 82.5% yield;
¹H NMR (400 MHz, CDCl₃) δ = 3.80 (s, 3H), 6.88 (d, J = 8Hz, 1H), 6.92 (t, J = 8Hz, 1H), 7.03 (t, J = 7.2Hz, 1H), 7.16 (m, 2H), 7.34 (d, J = 7.2Hz, 1H), 7.68 (d, J = 8 Hz, 1H), 7.89 (d, J = 7.2 Hz, 1H), 8.12 (d, J = 8Hz, 2H), 8.43 (s, 1H), 8.57 (s, 1H) ppm.
ESI-MS: [M+Na]⁺ m/z 448.

### Example 2

### General Procedure for 2-amino-N-(2-methoxyphenyl)-6-(4-nitrophenyl-thio)benzamide hydrochloride

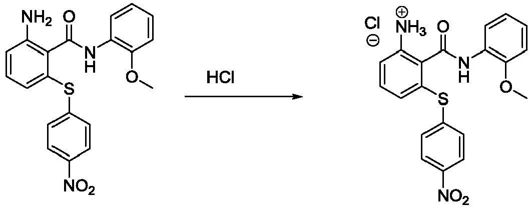

2-amino-N-(2-methoxyphenyl)-6-(4-nitrophenyl-thio)benzamide dissolved in EA, stirred at room temperature, while HCl is inlet till occurred to white solid. The reaction mixture was filtered to get White solid; 94.2% yield;
¹H NMR (400 MHz, DMSO) δ = 3.69 (s, 3H), 5.33 (br, 3H), 6.81 (d, J = 7.6Hz, 1H), 6.92 (m, 2H), 7.01 (d, J = 8Hz, 1H), 7.12 (t, J = 7.6Hz, 1H), 7.25 (t, J = 8Hz, 1H), 7.31 (d, J = 8.8Hz, 2H), 7.758 (d, J = 8Hz, 1H), 8.098 (d, J = 8.4Hz, 2H), 9.397 (br, 1H) ppm.
ESI-MS: [M+Na]⁺ m/z 403.

### Example 3

### General Procedure for 2-amino-N-(2-methoxyphenyl)-6-(4-nitrophenyl-thio)benzamide sulfate

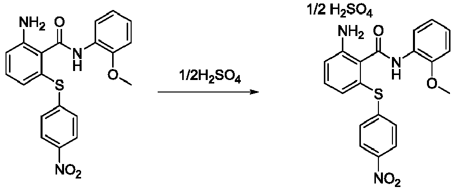

2-amino-N-(2-methoxyphenyl)-6-(4-nitrophenyl-thio)benzamide dissolved in EA, stirred at room temperature, while dropped H₂SO₄ into mixture till occurred to white solid. The reaction mixture was filtered to get White solid; 72% yield;
¹H NMR (400 MHz, DMSO) δ = 3.69 (s, 3H), 4.02 (br, 3H), 6.78 (d, J = 8.4Hz, 1H), 6.90 (m, 2H), 7.01 (d, J = 7.6Hz, 1H), 7.10 (t, J = 7.6Hz, 1H), 7.236 (t, J = 8Hz, 1H), 7.305 (d, J = 8.8Hz, 2H), 7.753 (d, J = 7.6Hz, 1H), 8.097 (d, J = 8.8Hz, 2H), 9.379 (br, 1H) ppm.
ESI-MS: [M+Na]⁺ m/z 448.

### Comparative Example 3

### General Procedure for 2-amino-N-(2-methoxyphenyl)-6-((4-nitrophen-yl)sulfinyl)benzamide

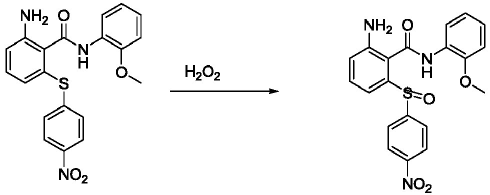

Compound **25** (1.0 equiv.) was dissolved in CH₃OH (30 mL) with a magnetic stirrer at RT, and 35%H₂O₂ (3.0 equiv.) was added slowly to this solution. This reaction mixture was heated in an oil bath at 66°C and monitored using TLC. The reaction was quenched with MnO₂ at RT. The mixture was filtered and the residue was washed with ethyl acetate (3 × 5 mL). The liquid layer was concentrated under reduced pressure and the oily residue was purified by silica gel column chromatography (1:4 v/v ethyl acetate/petroleum ether) to provide a yellow solid; 44.3% yield;
¹H NMR (400 MHz, CDCl₃) *δ* = 3.90 (s, 3H), 4.46 (brs, 2H), 6.88 (d, *J* = 8.0Hz, 1H), 6.96 (d, *J* = 8.0Hz, 1H), 7.04 (t, *J* = 7.6Hz, 1H), 7.17 (t, *J* = 8.0Hz, 1H), 7.41 (t, *J* = 8.0Hz, 1H), 7.47 (d, *J* = 7.2Hz, 1H), 7.91 (d, *J* = 8.8Hz, 2H), 8.21 (d, *J* = 8.8Hz, 2H), 8.28 (d, *J* = 7.2Hz, 1H), 8.75 (brs, 1H).
ESI-MS: [M+Na]⁺ m/z 434.

### Example 4

### General Procedure for 2-amino-N-(2-methoxyphenyl)-6-((4-nitroph-enyl)sulfonyl)benzamide (compound 46)

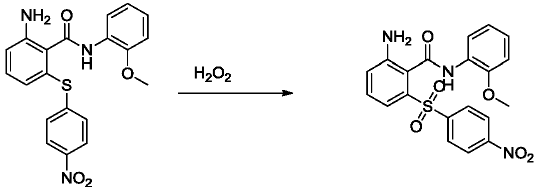

Compound 25 (1.0 equiv.) was dissolved in acetic acid (30 mL) with a magnetic stirrer at RT, and H₂O₂ (3.0 equiv.) was added to this solution. This reaction mixture was heated in an oil bath at 65 °C and the reaction was monitored using TLC. The reaction was quenched with MnO2 (3.5 equiv.) at RT. The mixture was filtered and the residue was washed with ethyl acetate (3 × 10 mL). The liquid layer was concentrated under reduced pressure and the oily residue was purified by silica gel column chromatography (1:2 v/v ethyl acetate/petroleum ether) to provide the product as a yellow amorphous solid (162.4 mg, 38% yield).
¹H NMR (400 MHz, DMSO) δ = 3.79 (s, 3H), 5.57 (s, 2H), 7.01 (t, J = 7.6Hz, 1H), 7.08 (m, 2H), 7.22 (t, J = 7.6Hz, 1H), 7.29 (d, J = 7.6Hz, 1H), 7.36 (t, J = 7.6Hz, 1H), 7.76 (d, J = 7.6Hz, 1H), 8.16 (d, J = 8.8Hz, 2H), 8.36 (d, J = 8.8Hz, 2H), 9.71 (s, 1H).
ESI-MS: [M+H]⁺m/z 428.

### Comparative Example 4

### General Procedure for 5-amino-N-(2-methoxyphenyl)-2-((4-nitrophenyl)thio)benzamide (compound 24)

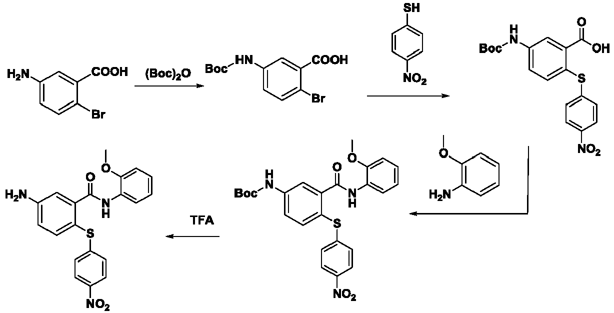

### Intermediate 4a

### Synthesis of 2-bromo-5-(tert-butoxycarbonylamino)benzoic acid

A mixture of 2-bromo-5-aminobenzoate (1.0 equiv.), BOC anhydride (1 equiv.) was dissolved in 15 mL THF, and stirring at room temperature, and DMAP (0.5 equiv.) was added to this solution, then dropped triethylamine (2.0 equiv.) to the mixture. The reaction stirring for 5 hours at room temperature. After the reaction was complete, the reaction solution was concentrated under reduced pressure, concentrate was dissolved in ethyl acetate, the organic layer was washed once with saturated brine, dried over anhydrous Na₂SO₄, purified by column chromatography (petroleum ether: ethyl acetate) to give a yellow solid 8a, in 95% yield.

### Intermediate 4b

### Synthesis of 5-(tert-butoxycarbonylamino)-2-(4-nitrophenyl-thio)benzoic acid

A mixture of intermediate 8a (1.0 equiv.), 4-nitrobenzenethiol (2.0 equiv.), nanometer copper powder (0.5 equiv.) and anhydrous potassium carbonate (3.0 equiv.) were mixed in DMF (40 mL) at RT. Then the reaction flask was heated in an oil bath at 55°C. After 8h, the heating bath was removed and the reaction flask was allowed to cool to room temperature. The reaction mixture was filtered and the liquid layer was diluted with water (40 mL), extracted with ethyl acetate (3 × 20 mL), washed with water (6 × 15 mL), dried over anhydrous Na₂SO₄, filtered, and concentrated under reduced pressure. The residue was purified by silica gel column chromatography (1:3 v/v ethyl acetate/petroleum ether) to provide the product intermediate 8b as a yellow amorphous solid; 77% yield.

### Intermediate 4c

### Synthesis of tert-butyl 3-(2-methoxyphenylcarbamoyl)-4-(4-nitrophenylthio)phenylcarbamate

A mixture of 2-methoxyaniline(1.0 equiv.), intermediate 8b (1.0 equiv.), EDCI(1.2 equiv.) in DCM(10ml) was stirred at room temperature for 5 hrs. After the reaction was completed, the DCM was removed by vaccum distillation. The resulting reaction mixture was extracted with EtOAc (3x20 mL). The combined oiganic extracts were washed with water (20 mL), brine (20 mL), dried over MgSO4 and concentrated in vacuo. The residue was purified over silica gel using EtOAc:Hexanes (1:4) astheeluent to afford intermediate 8c (yield 79%) as a white solid.

### Synthesis of 5-amino-N-(2-methoxyphenyl)-2-(4-nitrophenylthio)benzamide

The 8c (1.0 equiv.) was dissolved in DCM, stirring at room temperature, dropwise trifluoroacetic acid(TFA 5 mmol) into the mixtuer, then stirred for 2 hours at room temperature. After completion of the reaction, the reaction solution was concentrated under reduced pressure, concentrate was dissolved in ethyl acetate, the organic layer was washed once with saturated brine, dried over anhydrous sodium sulfate and then was added, and finally the organic layer was concentrated under reduced pressure, purified by column chromatography (petroleum ether: ethyl acetate) to give a yellow solid, yield 84%.
¹H NMR (400 MHz, CDCl₃) *δ* = 3.71 (s, 3H), 4.20 (brs, 2H), 6.82 (m, 2H), 6.95 (t, *J* = 7.6Hz, 1H), 7.04 (t, *J* = 8.0Hz, 1H), 7.14 (m, 3H), 7.42 (d, *J* = 8.0Hz, 1H), 8.03 (d, *J* = 9.2Hz, 2H), 8.40 (d, *J* = 8.0Hz, 1H), 8.45 (brs, 1H).
ESI-MS: [M+H]⁺ m/z 396.

### Comparative Example 5

### General Procedure for N-(2-methoxyphenyl)-5-(methylamino)-2-((4-nitrophenyl)thio)benzamide (compound 27)

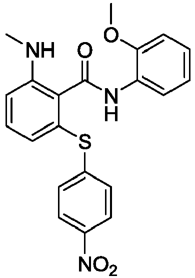

Compound 24 (1.0 equiv.) was dissolved in DMF (40 mL) and treated with anhydrous potassium carbonate (2.0 equiv.) at room temperature. To this mixture was added iodomethane (0.9 equiv.) via a micro syringe. After being stirred for 10h at 50 °C, the reaction mixture was poured into ice-cold water (200 mL) to get crude product. The crude solid was filtered and washed several times with water and purified by silica gel column chromatography (1:6 v/v ethyl acetate/petroleum ether) to provide a yellow solid (43% yield).
¹H NMR (400 MHz, CDCl₃) δ = 2.93 (s, 3H), 3.69 (s, 3H), 4.24 (brs, 1H), 7.72 (d, J = 8.4Hz, 1H), 6.82 (d, J = 8.0Hz, 1H), 6.94 (t, J = 6.4Hz, 1H), 7.03 (m, 2H), 7.13 (d, J = 8.8Hz, 2H), 7.43 (d, J = 8.4Hz, 1H), 8.02 (d, J = 8.8Hz, 2H), 8.41 (d, J = 8.0Hz, 1H), 8.45 (brs, 1H).
ESI-MS: [M+H]⁺ m/z 410.

### Comparative Example 6

### General Procedure for 5-(dimethylamino)-N-(2-methoxyphenyl)-2-((4-nitrophenyl)thio)benzamide (compound 28)

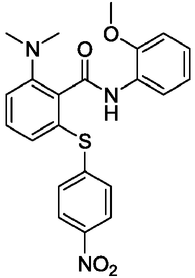

Compound 24 (1.0 equiv.) was dissolved in DMF (40 mL) and treated with anhydrous potassium carbonate (4.0 equiv.) at room temperature. To this mixture was added iodomethane (2.5 equiv.) via a micro syringe. After being stirred for 10Hours at 60 °C, the reaction mixture was poured into ice-cold water (200 mL) to get crude product. The crude solid was filtered and washed several times with water and purified by silica gel column chromatography (1:8 v/v ethyl acetate/petroleum ether) to provide a yellow solid (78.1% yield).
1H NMR (400 MHz, CDCl₃) δ = 3.09 (s, 6H), 3.69 (s, 3H), 6.83 (m, 2H), 6.95 (t, J = 6.8Hz, 1H), 7.04 (t, J = 7.6Hz, 1H), 7.13 (m, 3H), 7.47 (d, J = 8.4Hz, 1H), 8.03 (d, J = 9.2Hz, 2H), 8.43 (dd, J = 8.0,1.2Hz, 1H), 8.48 (brs, 1H).
ESI-MS: [M+H]⁺ m/z 424.

### Comparative Example 7

### General Procedure for 5-acetamido-N-(2-methoxyphenyl)-2-((4-nitrophenyl)thio) benzamide (compound 29)

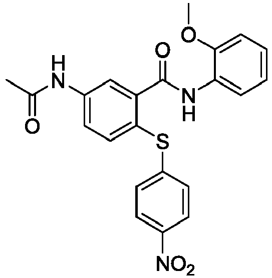

Compound 24 (1.0 equiv.) was dissolved in DCM (30 mL) at room temperature. To this mixture was added acetic anhydride (1.2 equiv.), followed by the addition of DMAP (0.5 equiv.). After being stirred for 5 hours at room temperature, the reaction mixture was concentrated under reduced pressure. The crude residue was diluted ethyl acetate (60 mL), washed with water (2 × 15 mL), dried over anhydrous Na₂SO₄, filtered, and concentrated under reduced pressure. The residue was purified by silica gel column chromatography (1:6 v/v ethyl acetate/petroleum ether) to provide a yellow solid; 96% yield;
¹H NMR (400 MHz, CDCl₃) *δ* = 2.18 (s, 3H), 3.73 (s, 3H), 6.85 (d, *J* = 8.4Hz, 1H), 6.95 (t, *J* = 7.6Hz, 1H), 7.07 (t, *J* = 8.0Hz, 1H), 7.19 (d, *J* = 8.8Hz, 2H), 7.56 (d, *J* = 8.8Hz, 1H), 7.83 (d, *J* = 1.6Hz, 1H), 7.94 (d, *J* = 8.0Hz, 1H), 7.98 (br, 1H), 8.04 (d, *J* = 9.2Hz, 2H), 8.35 (d, *J* = 7.6Hz, 1H), 8.51 (br, 1H) ppm.
ESI-MS: [M+H]⁺*m*/*z* 438.

### Example 5

### General Procedure for 2-amino-N-(2-methoxyphenyl)-6-(4-nitrop-henoxy)benzamide (compound 48)

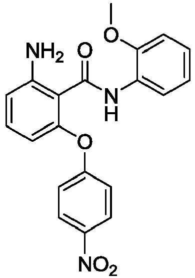

Prepared by proceeding in similar manner to example 1, use 4-Nitrophenol instead of 4-nitrothiophenol. Yellow solid; 38% yield;
¹H NMR (400 MHz, CDCl₃) *δ* = 3.77 (s, 3 H), 5.88 (br, 2 H), 6.28 (d, J = 8 Hz, 1 H), 6.60 (d, J = 8 Hz, 1 H), 6.90 (m, 2 H), 7.67 (t, *J* = 8 Hz, 1 H), 7.00 (d, *J* = 8 Hz, 2 H), 7.18 (t, *J* = 8 Hz, 1 H), 8.21 (d, *J* = 8.8 Hz, 2 H), 8.42 (d, *J* = 8 Hz, 1 H), 9.49 (s, 1 H)ppm.
ESI-MS: [M+Na]⁺ *m*/*z* 402.

### Comparative Example 8

### General Procedure for 2-amino-N-(3-aminophenyl)-6-(4-nitroph-enylthio)benzamide (compound 49)

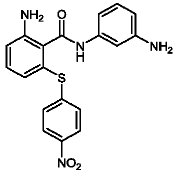

Prepared by proceeding in similar manner to example 1, use m-phenylenediamine istead of o-anisidine. Yellow solid; 38% yield;
¹H NMR (400 MHz, CDCl₃) *δ* = 3.62 (br, 2 H), 5.78 (br, 2 H), 6.31 (d, *J* = 8.4 Hz, 1 H), 6.62 (d, *J* = 7.6 Hz, 1 H), 7.01 (m, 1 H), 7.45 (d, *J* = 8 Hz, 2 H), 7.48 (m, 2 H), 7.82 (d, J = 8 Hz, 1 H), 7.93 (d, J = 8.8 Hz, 2 H), 8.01 (d, J = 7.2 Hz, 1 H), 8.52 (s, 1H)ppm.
ESI-MS: [M+K]⁺ m/z 418.

### Comparative Example 9

### General Procedure for 2-amino-N-(4-amino-2-methoxyphenyl)-6-(4-nitrophenylthio) benzamide (compound 50)

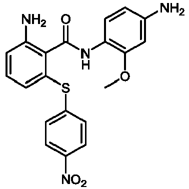

Prepared by proceeding in similar manner to example 1, use 2-methoxybenzene -1,4-diamine instead of o-anisidine. Yellow solid; 69.8% yield;
1H NMR (400 MHz, CDCl₃) δ = 3.82 (s, 3 H), 5.66 (br, 2 H), 6.42 (d, J = 8 Hz, 2 H), 6.67 (t, J = 7.2 Hz, 2 H), 7.12 (t, J = 8 Hz, 1 H), 7.56 (m, 4 H), 8.00 (s, 1 H), 8.33 (d, J = 8 Hz, 1 H), 8.92 (br, 2 H)ppm.
ESI-MS: [M+H]⁺ m/z 411.

### Comparative Example 10

### General Procedure for 2-amino-N-(2,4-dimethoxyphenyl)-6-(4-nitrophenylthio) benzamide(compound 51)

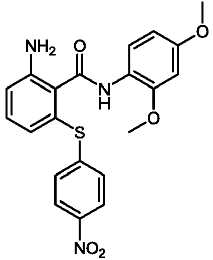

Prepared by proceeding in similar manner to example 1, use 2,4-dimethoxyaniline instead of o-anisidine. 61% yield;
1H NMR (400 MHz, CDCl₃) δ = 3.82 (s, 3 H), 3.84 (s, 3 H), 6.52 (d, J = 9.6 Hz, 2 H), 6.69 (t, J = 9.2 Hz, 2 H), 7.08 (t, J = 8 Hz, 1 H), 7.65 (m, 4 H), 8.15 (s, 1 H), 8.29 (d, J = 8.8 Hz, 1 H), 9.05 (br, 2 H)ppm.
ESI-MS: [M+K]⁺ m/z 474.

### Comparative Example 11

### General Procedure for N-(2-methoxyphenyl)-2-nitro-6-(4-nitrophenylthio)benzamide (compound 52)

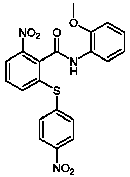

Prepared by proceeding in similar manner to example 1, use 2-bromo-6-nitrobenzoic acid instead of 2-amino-6-bromobenzoic acid. 77% yield;
¹H NMR (400 MHz, CDCl₃) *δ* = 3.73 (s, 3 H), 6.85 (d, *J* = 7.2 Hz, 1 H), 7.00 (t, *J* = 8 Hz, 1 H), 7.10 (t, *J* = 8.8 Hz, 1 H), 7.30 (d, *J*=8.8 Hz, 2 H), 7.66 (t, *J*=8 Hz, 1 H), 7.85 (d, *J*=8.8 Hz, 2 H), 8.09 (d, *J*=9.2 Hz, 2 H), 8.28 (d, *J*=7.2 Hz, 1 H), 6.85 (d, *J*=7.2 Hz, 1 H)ppm.
ESI-MS: [M+K]⁺*m*/*z* 474.

### Comparative Example 12

### General Procedure for N-(2-aminophenyl)-2-nitro-6-(4-nitrophenylthio)benzamide (compound 53)

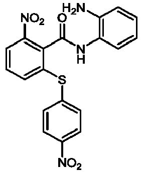

Prepared by proceeding in similar manner to example 16, use o-Phenylenediam-ine instead of o-anisidine. 68.5% yield;
¹H NMR (400 MHz, CDCl₃) *δ* = 3.57 (br, 2 H), 6.83 (d, *J* = 8 Hz, 2 H), 7.14 (t, *J* = 7.2 Hz, 1 H), 7.22 (d, *J* = 8 Hz, 2 H), 7.40 (d, *J* = 8 Hz, 2 H), 7.68 (t, *J* = 7.6 Hz, 1 H), 7.85 (d, *J* = 8 Hz, 1 H), 7.09 (d, *J* = 8 Hz, 2 H), 8.31 (d, *J* = 8 Hz, 1 H)ppm.
ESI-MS: [M+H]⁺*m*/*z* 411.

### Comparative Example 13

### General Procedure for 3-(2-nitro-6-(4-nitrophenylthio) benzamildo)benzoic acid (compound 54)

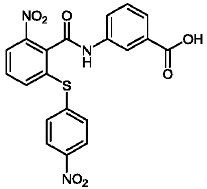

Prepared by proceeding in similar manner to example 16, use 3-aminobenzoic acid instead of o-anisidine. 43.1% yield;
¹H NMR (400 MHz, DMSO) *δ* =7.43 (d, *J* = 8.8 Hz, 2 H), 7.48 (d, *J* = 8 Hz, 1 H), 7.71 (m, 2 H), 7.84 (t, *J* = 8 Hz, 1 H), 8.03 (d, *J* = 8 Hz, 1 H), 8.15 (d, *J* = 8.8 Hz, 2 H), 8.21 (s, 1 H), 8.39 (d, *J* = 8 Hz, 1 H), 10.89 (s, 1 H), 13.04 (br, 1 H)ppm.
ESI-MS: [M-H]⁻ *m*/*z* 438.

### Comparative Example 14

### General Procedur for N-(2-methoxyphenyl)-2-(4-nitrophenylthio)-6-(phenylsulfonamido) benzamide(compound 56)

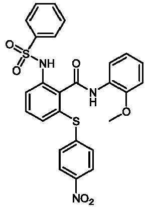

Compound **25** (1.0 equiv.) was dissolved in azabenzene (40 mL) at room temperature. To this mixture was added benzenesulfonyl chloride (1.2 equiv.). After being stirred for 10Hours at room temperature, the reaction mixture was concentrated under reduced pressure. The crude residue was diluted ethyl acetate (50 mL), washed with water (3 × 20 mL), dried over anhydrous Na₂SO₄, filtered, and concentrated under reduced pressure. The residue was purified by silica gel column chromatography (1:4 v/v ethyl acetate/petroleum ether) to provide a white solid. 67% yield;.
¹H NMR (400 MHz, CDCl₃) *δ* = 3.65 (s, 3H), 6.74 (d, *J* = 8.0Hz, 1H), 6.85 (t, *J* = 7.6Hz, 1H), 6.91 (d, *J* = 8.0Hz, 1H), 7.02 (t, *J* = 7.6Hz, 1H), 7.28 (m, 1H), 7.37 (t, *J* = 8.0Hz, 1H), 7.47 (t, *J* = 7.6Hz, 3H), 7.64 (m, 3H), 8.01 (m, 5H), 8.93 (s, 1H) ppm.
ESI-MS: [M+K]⁺ *m*/*z* 558.

### Comparative Example 15

### General Procedure for N-(2-methoxyphenyl)-2-(4-methylphenylsulfonamido)-6-((4-nitrophenyl)thio) benzamide (compound 57)

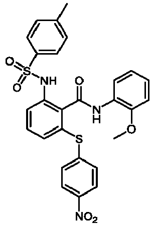

Prepared by proceeding in similar manner to example 20, using Methyl-benzenes-ulfonyl chloride instead of benzenesulfonyl chloride. White solid; 83.8% yield;
¹H NMR (400 MHz, CDCl₃) *δ* = 2.17 (s, 3H), 3.69 (s, 3H), 6.8 (d, *J* = 8Hz, 1H), 6.95 (d, *J* = 8.8Hz, 3H), 7.09 (m, 3H), 7.38 (d, *J* = 8.0Hz, 1H), 7.47 (t, *J* = 8.0Hz, 1H), 7.54 (d, *J* = 8.0Hz, 2H), 7.81 (d, *J =* 8.0Hz, 1H), 7.95 (s, 1H), 8.01 (d, *J* = 8.8Hz, 2H), 8.16 (d, *J* = 8.0Hz, 1H), 8.42 (s, 1H);
ESI-MS: [M+Na]⁺ m/z 572.09

### Comparative Example 16

### General Procedure for 2-(dimethylamino)-N-(2-methoxyphenyl)-6-((4-nitrophenyl) sulfonyl)benzamide (compound 58)

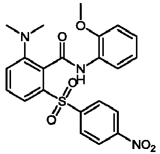

Prepared by proceeding in similar manner to example 7, using compound 28 instead of compound 25.Yellow solid; 77.2% yield;
¹H NMR (400MHz, CDCl₃) *δ* = 2.76 (s, 3H), 3.28 (s, 3H), 4.08 (s, 3H), 6.61 (d, *J* = 8.0Hz, 1H), 7.09 (t, *J* = 7.6Hz, 1H), 7.13 (d, *J* = 8.0Hz, 1H), 7.21 (d, *J* = 7.6Hz, 2H), 7.36 (m, 2H), 7.43 (t, *J* = 6.4Hz, 1H), 7.60 (d, *J* = 9.2Hz, 2H), 7.91 (d, *J* = 8.0Hz, 2H);
ESI-MS: [M+Na]⁺ m/z 478.10

### Comparative Example 17

### General Procedure for N-(2-methoxyphenyl)-2-(methylamino)-6-((4-nitrophenyl) sulfonyl)benzamide (compound 59)

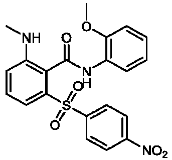

Prepared by proceeding in similar manner to example 7, using compound 27 instead of compound 25.White solid; 85.2% yield;
¹H NMR (400 MHz, CDCl₃) *δ* = 3.29 (s, 3H), 4.03 (s, 3H), 4.73 (brs, 1H), 7.21 (dd, *J* = 8.0,0.8Hz, 1H), 7.04 (td, *J* = 8.0,1.2Hz, 1H), 7.15 (dd, *J* = 8.0,1.2Hz, 1H), 7.24 (m, 3H), 7.41 (m, 2H), 7.57 (m, 2H), 7.95 (d, *J* = 12.0Hz, 2H);
ESI-MS: [M+Na]⁺ m/z 464.08

### Comparative Example 18

### General Procedure for N-(2-methoxyphenyl)-2-(4-methylphenyls-ulfonamido)-6-((4-nitrophenyl)sulfonyl)benzamide (compound 60)

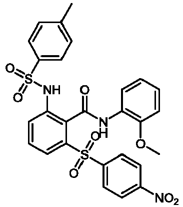

Prepared by proceeding in similar manner to example 7, using compound 57 instead of compound 25, White solid; 55% yield;
¹H NMR (400 MHz, CDCl₃) *δ* = 2.17 (s, 3H), 3.64 (s, 3H), 6.77 (d, *J* = 8.0Hz, 1H), 6.95 (m, 2H), 7.09 (t, *J* = 7.2Hz, 1H), 7.19 (d, *J* = 8.0Hz, 1H), 7.42 (t, *J* = 7.2Hz, 2H), 7.61 (t, *J* = 7.2Hz, 1H), 7.81 (d, *J* = 7.6Hz, 1H), 7.95 (d, *J* = 7.6Hz, 2H), 8.10 (d, *J* = 8.0Hz, 1H), 8.26 (d, *J* = 8.4Hz, 2H), 8.33 (d, *J* = 8.4Hz, 2H), 9.18 (s, 1H);
ESI-MS: [M+Na]⁺ m/z 604.08

### Comparative Example 19

### General Procedure for N-(2-methoxyphenyl)-2-((4-nitrophenyl)sulfonyl)-6-(phenylsulfonamido)benzamide (compound 61)

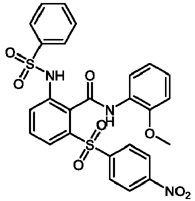

Prepared by proceeding in similar manner to example 7, using compound 56 instead of compound 25, White solid; 62% yield;
¹H NMR (400 MHz, CDCl₃) *δ* = 3.64 (s, 3H), 6.77 (d, *J* = 7.2Hz, 1H), 6.95 (m, 2H), 7.09 (t, *J* = 8.0Hz, 1H), 7.44 (t, *J* = 8.0Hz, 2H), 7.49 (t, *J* = 7.2Hz, 1H), 7.61 (t, *J* = 7.2Hz, 2H), 7.95 (d, *J* = 7.2Hz, 3H), 8.10 (d, *J* = 9.2Hz, 1H), 8.27 (d, *J* = 7.2Hz, 2H), 8.32 (d, *J* = 7.2Hz, 2H), 9.18 (s, 1H);
ESI-MS: [M+Na]⁺ m/z 590.06

### Comparative Example 20

### General Procedure for N-(2-methoxyphenyl)-2-((4-nitrophenyl)thio)-6-(piperazinyl) benzamide (compound 62)

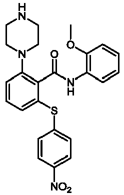

Compound **25** (1.0 equiv.) was dissolved in DMF (40 mL) at room temperature. To this mixture bis(2-bromoethyl)amine (1.0 equiv.) was added, following the addition of NaOH (2.0 equiv.). After being stirred for 2Hours at room temperature, the reaction mixture was poured into ice-cold water (200 mL) to get crude product. The crude solid was filtered and washed several times with water and purified by silica gel column chromatography (1:3 v/v ethyl acetate/petroleum ether) to provide a yellow solid (380.9 mg, 82% yield).
¹H NMR (400 MHz, CDCl₃) *δ* = 1.11 (s, 2H), 1.60 (s, 2H), 2.48 (t, *J* = 4.8Hz, 2H), 3.13 (t, *J* = 6.0Hz, 2H), 3.81 (s, 3H), 5.30 (s, 1H), 6.91 (d, *J* = 8.0Hz, 1H), 7.02 (d, *J* = 8.0Hz, 3H), 7.13 (t, *J* = 7.6Hz, 1H), 7.32 (m, 2H), 7.88 (s, 1H), 8.11 (d, *J* = 8.8Hz, 2H), 8.36 (d, *J* = 8.0Hz, 1H), 9.46 (s, 1H);
ESI-MS: [M+H]⁺ m/z 465.16

### Comparative Example 21

### General Procedure for N-(2-methoxyphenyl)-2-morpholino-6-((4-nitrophenyl)thio) benzamide (compound 63)

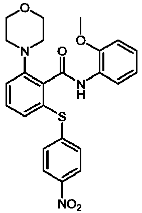

Prepared by proceeding in similar manner to example 26, using 2,2'-dibromodi-ethyl ether instead of bis(2-bromoethyl)amine, Yellow solid; 67% yield;
¹H NMR (400 MHz, CDCl₃) *δ* = 1.33 (s, 2H), 1.74 (s, 2H), 2.55 (t, *J* = 4.8Hz, 2H), 3.21 (t, *J =* 6.0Hz, 2H), 3.82 (s, 3H), 7.02 (d, *J* = 7.2Hz, 1H), 7.12 (d, *J* = 8.8Hz, 3H), 7.23 (t, *J* = 7.2Hz, 1H), 7.32 (m, 2H), 7.91 (s, 1H), 8.22 (d, *J* = 8.0Hz, 2H), 8.32 (d, *J* = 8.0Hz, 1H), 9.46 (s, 1H);
ESI-MS: [M+H]⁺ m/z 466.14

### Comparative Example 22

### General Procedure for 2-((2-bromoethyl)amino)-N-(2-methoxyp-henyl)-6-((4-nitrophenyl)thio)benzamide (compound 64)

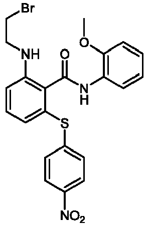

Prepared by proceeding in similar manner to example 9, Yellow solid; 59.5% yield;
¹H NMR (400 MHz, CDCl₃) *δ* = 3.27 (t, *J* = 7.2Hz, 2H), 3.45 (m, 2H), 3.87 (s, 3H), 4.14 (brs, 1H), 6.49 (d, *J* = 9.2Hz, 1H), 6.68 (d, *J* = 4.0Hz, 1H), 7.04 (m, 3H), 7.37 (m, 3H), 7.53 (t, *J* = 4.0Hz, 1H), 7.96 (d, *J* = 8.0Hz, 2H), 8.20 (s, 1H);
ESI-MS: [M+H]⁺ m/z 502.04

### Comparative Example 23

### General Procedure for diisopropyl(((2-((2-methoxyphenyl)carbamoyl)-3-((4-nitro-phenyl)thio)phenyl)amino)methyl)phosphonate (compound 65)

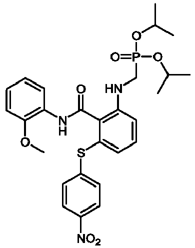

Compound **25** (1.0 equiv.) was dissolved in MeCN (40 mL) and treated with anhydrous potassium carbonate (2.0 equiv.) at room temperature. To this mixture diisopropyl (bromomethyl) phosphonate (1.2 equiv.) was added via a micro syringe. After being stirred for 4Hours at 60 °C, the reaction mixture was concentrated under reduced pressure. The crude residue was diluted by ethyl acetate (50 mL), and washed with water (2 × 10 mL). Then it was dried over anhydrous Na₂SO₄, filtered, and concentrated under reduced pressure. The residue was purified by silica gel column chromatography (1:4 v/v ethyl acetate/petroleum ether) to provide a yellow solid (235.2 mg, 41% yield).
¹H NMR (400 MHz, DMSO) *δ* = 1.28 (m, 12H), 3.33 (s, 3H), 3.83 (m, 2H), 5.31 (brs, 1H), 6.39 (d, *J* = 8.0Hz, 1H), 6.71 (t, *J* = 7.6Hz, 2H), 7.04 (t, *J* = 7.6Hz, 1H), 7.42 (m, 3H), 7.55 (d, *J* = 8.0Hz, 1H), 7.88 (d, *J* = 8.0Hz, 2H), 8.03 (d, *J* = 8.0Hz, 1H), 8.56 (s, 1H);
ESI-MS: [M+H]⁺ m/z574.18

### Example 6

### General Procedure for 2-(2-aminoacetamido)-N-(2-methoxyphenyl)-6-(4-nitrophenylthio) benzamide hydrochloride (compound 66)

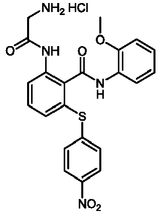

The compound 25 (1.0 equiv.), HOBt (1.2 equiv.) and EDCI (1.2 equiv.) were dissolved in DCM, stirring at room temperature. After that, glycine (1.0 equiv.) was slowly added into the mixture, stirring for 5 h. Then the reaction solution was concentrated under reduced pressure, and the concentrate was dissolved in ethyl acetate. The organic layer was once washed with brine, and dried over anhydrous Na₂SO₄. Then the organic layer was concentrated under reduced pressure. The concentrate with an appropriate amount of HCl was stirred for 2 hours, and filtered. The filter cake was washed with ethanol once and then washed with DCM to give a white solid; yield 45%;

¹H NMR (400MHz, DMSO): 3.244 (s, 2 H), 3.684 (s, 3 H), 4.941 Cbr, 2 H), 6.915 (t, *J*=7.6 Hz, 1 H), 7.015 (d, *J*=8 Hz, 1 H), 7.131 (t, *J*=7.2 Hz, 1 H), 7.343 (m, 3 H), 7.548 (t, *J*=8 Hz, 1 H), 7.875 (d, *J*=8Hz, 1 H), 8.111 (d, *J*=8.8 Hz, 2 H), 8.412 (d, *J*=8Hz, 1H), 9.904 (s, 1 H) ppm.
ESI-MS: [M+U]⁺ *m*/*z* 453.1.

### Example 7

### General Procedure for 2-(2-aminoacetamido)-N-(2-methoxyphenyl)-6-((4-nitrophenyl) sulfonyl)benzamide hydrochloride hydrochloride (compound 67)

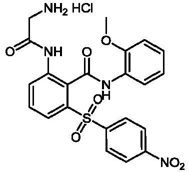

Prepared by proceeding in similar manner to example 30, use compound 46 instead of compound 25. White solid; 51 % yield;
¹H NMR (400 MHz, DMSO) *δ* = 3.73 (S, 2H), 3.82 (S, 3H), 7.02 (t, *J* = 6.8 Hz, 1H), 7.09 (d, *J* = 7.6 Hz, 1H), 7.19 (t, *J* =7.2 Hz, 1H), 7.77 (t, *J* = 6.8 Hz, 1H), 8.01 (t, *J* = 7.6 Hz, 2H), 8.08 (d, *J* = 7.6 Hz, 1H), 8.23 (d, *J* = 8.4 Hz, 2H), 8.39 (m, 4H), 9.89 (S, 1H), 10.00 (br, 2H) ppm.
ESI-MS: [M+H]⁺ m/z 485.

### Example 8

### General Procedure for 2-(2,6-diaminohexanamido)-N-(2-methoxyphenyl)-6-((4-nitrophenyl)thio)benzamide hydrochloride (compound 68)

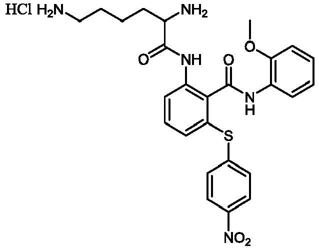

Prepared by proceeding in similar manner to example 30, use lysine of glycine. white solid; 39% yield;
¹H NMR (400 MHz, DMSO) *δ* = 1.39 (m, 8H), 1.68 (S, 1H), 3.68 (S, 3H), 6.49 (S, 1H), 6.92 (t, *J* =7.6 Hz, 1H), 7.01 (d, *J* = 8.0 Hz, 1H), 7.12 (t, *J* = 7.6 Hz, 1H), 7.34 (m, 3H), 7.54 (t, *J* = 7.6 Hz, 1H), 7.88 (d, *J* = 7.6Hz, 1H), 8.11 (d, *J* = 8.0 Hz, 2H), 8.34 (d, *J* = 8.0 Hz, 1H), 9.89 (S, 1H) ppm.
ESI-MS: [M+H]⁺ m/z 524.

### Comparative Example 24

### General Procedure for 2-amino-N-(2-iodophenyl)-6-((4-nitrophenyl)thio)benzamide (compound 71)

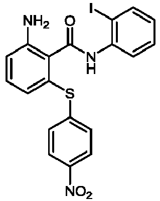

Prepared by proceeding in similar manner to example 1, Yellow solid; 79% yield;
¹H NMR (400 MHz, CDCl₃) *δ* = 4.61 (br, 2H), 6.86 (t, *J* = 7.6 Hz, 2H), 6.99 (d, *J* = 7.6 Hz, 1H), 7.28 (m, 4H), 7.78 (d, *J* = 7.6 Hz, 1H), 8.03 (d, *J* = 7.2 Hz, 3H), 8.11 (d, *J* = 8.0 Hz, 1H) ppm.
ESI-MS: [M+Na]⁺m/z 514.

### Comparative Example 25

### General Procedure for 2-amino-N-(2-iodophenyl)-6-((4-nitrophenyl)sulfonyl)benzamide (compound 72)

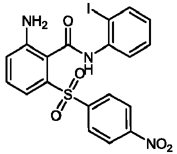

Prepared by proceeding in similar manner to example 7, White solid; 51.4% yield;
¹H NMR (400 MHz, CDCl₃) *δ* = 4.36 (br, 2H), 6.99 (m, 2H), 7.40 (t, *J* = 8.0 Hz, 1H), 7.46 (t, *J* = 7.2 Hz, 1H), 7.53 (d, *J* = 7.6 Hz, 1H), 7.78 (s, 1H), 7.86 (d, *J* = 7.6 Hz, 1H), 8.13 (d, *J* = 7.6 Hz, 2H), 8.20 (d, *J* = 8.0 Hz, 1H), 8.30 (d, *J* = 8.0 Hz, 2H) ppm.
ESI-MS: [M+Na]⁺ m/z 546.

### Comparative Example 26

### General Procedure for 2-(2-amino-6-((4-nitrophenyl)thio)benzamildo)phenyl methanesulfonate (compound 73)

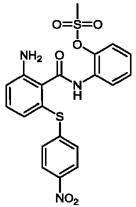

Prepared by proceeding in similar manner to example 1,Yellow solid; 56% yield;
¹H NMR (400 MHz, CDCl₃) *δ* = 3.13 (S, 3H), 3.22 (S, 3H), 4.48 (br, 2H), 6.83 (d, *J* = 8.0 Hz, 1H), 6.97 (d, *J* = 2.8 Hz, 1H), 7.19 (t, *J* = 7.2 Hz, 1H), 7.23 (m, 4H), 7.31 (t, *J* = 8.0 Hz, 1H), 8.05 (m, 1H), 8.35 (S, 1H) ppm.
ESI-MS: [M+Na]⁺ m/z 482.

### Comparative Example 27

### General Procedure for 2-amino-6-((3,5-dimethylphenyl)thio)-N-(2-methoxyphenyl) benzamide (compound 74)

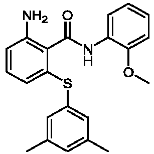

Prepared by proceeding in similar manner to example 1, White solid; 87.1% yield;
¹H NMR (400 MHz, CDCl₃) *δ* = 2.20 (S, 6H), 3.78 (s, 3H), 4.54 (br, 2H), 6.63 (d, *J* = 8.0 Hz, 1H), 7.69 (d, *J* = 8.0 Hz, 1H), 6.87 (m, 2H), 6.89 (S, 2H), 6.97 (t, *J* = 8.0 Hz, 1H), 7.08 (m, 2H), 8.42 (d, *J* = 8.0 Hz, 1H), 8.48 (s, 1H) ppm.
ESI-MS: [M+Na]⁺m/z 401.

### Comparative Example 28

### General Procedure for 2-amino-6-((3,5-dimethylphenyl)sulfonyl)-N-(2-methoxyphenyl) benzamide (compound 75)

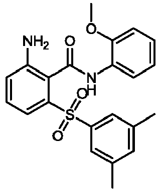

Prepared by proceeding in similar manner to example 7, Yellow solid; 62.8% yield;
¹H NMR (400 MHz, CDCl₃) *δ* = 2.19 (S, 6H), 3.84 (S, 3H), 4.31 (br, 2H), 6.92 (t, *J* = 8.0 Hz, 2H), 7.00(t, *J* = 8.0 Hz, 1H), 7.13 (m, 2H), 7.36 (m, 3H), 7.60 (d, *J* = 8.0 Hz, 1H), 8.31 (d, *J* = 8.0 Hz, 1H), 8.37 (S, 1H) ppm.
ESI-MS: [M+Na]⁺m/z 438.

### Comparative Example 29

### General Procedure for 4-((3-amino-2-((2-methoxyphenyl) carbamoyl)phenyl)thio) benzoic acid (compound 76

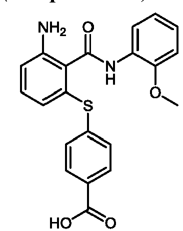

Prepared by proceeding in similar manner to example 1, Yellow solid; 36% yield;
¹H NMR (400 MHz, DMSO) *δ* = 3.82 (S, 3H), 5.36 (br, 2H), 6.91 (t, *J* = 8.0 Hz, 1H), 7.01 (d, *J* = 8.0 Hz, 1H), 7.15 (m, 2H), 7.24 (d, *J* = 8.0 Hz, 2H), 7.43 (d, *J* = 8.0 Hz, 1H), 7.60 (d, *J* = 8.0 Hz, 1H), 7.80 (d, *J* = 8.0 Hz, 2H), 8.21 (S, 2H) ppm.
ESI-MS: [M+Na]⁺m/z 417.

### Comparative Example 30

### General Procedure for 4-((3-amino-2-((2-methoxyphenyl) carbamoyl)phenyl)sulfonyl) benzoic acid (compound 77)

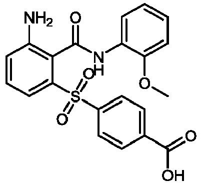

Prepared by proceeding in similar manner to example 7,Yellow solid; 22% yield;
¹H NMR (400 MHz, DMSO) *δ* = 3.78 (S, 3H), 5.55 (br, 2H), 7.03 (m, 2H), 7.09 (d, *J* = 8.0 Hz, 1H), 7.23 (m, 2H), 7.34 (t, *J* = 7.6 Hz, 1H), 7.77 (d, *J* = 7.6 Hz, 1H), 8.04 (m, 4H), 9.60 (S, 1H), 13.480 (br, 1H) ppm.
ESI-MS: [M+Na]⁺ m/z 449.

### Comparative Example 31

### General Procedure for 2-((2-aminoethyl)amino)-N-(2-methoxyphenyl)-6-((4-nitrophenyl) thio)benzamide (compound 78)

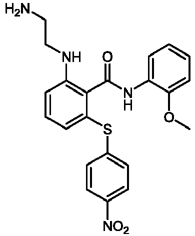

Prepared by proceeding in similar manner to example 9, Yellow solid; 51% yield;
¹H NMR (400 MHz, DMSO) *δ* = 0.82 (m, 2H), 1.18 (m, 2H), 2.00 (br, 2H), 3.78 (S, 3H), 6.35 (m, 3H), 6.67 (t, *J* = 7.6 Hz, 2H), 6.76 (m, 2H), 7.99 (d, *J* = 8.0 Hz, 1H), 7.16 (m, 3H), 7.44 (d, *J* = 8.0 Hz, 1H), 7.54 (S, 1H) ppm.
ESI-MS: [M+H]⁺ m/z 439.

### Comparative Example 32

### General Procedure for 2-((2-aminoethyl)amino)-N-(2-methoxyphenyl)-6-((4-nitrophenyl)sulfonyl) benzamide (compound 79)

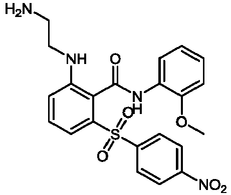

Prepared by proceeding in similar manner to example 7, Yellow solid; 49% yield;
¹H NMR (400 MHz, DMSO) *δ* = 0.85 (m, 2H), 1.26 (m, 2H), 2.09 (brs, 2H), 3.72 (s, 3H), 6.88 (d, *J* = 8.0Hz, 1H), 7.00 (m, 3H), 7.06 (s, 1H), 7.14 (t, *J* = 7.6Hz, 1H), 7.61 (t, *J* = 8.0Hz, 1H), 7.77 (d, *J* = 8.0Hz, 1H), 7.83 (d, *J* = 8.0Hz, 1H), 8.14 (d, *J* = 9.2Hz, 2H), 8.24 (d, *J* = 7.2Hz, 1H), 8.48 (s, 1H).
ESI-MS: [M+H]⁺ m/z 471.

### Comparative Example 33

### General Procedure for 2-((2-hydroxyethyl)amino)-N-(2-methoxyphenyl)-6-((4-nitrophenyl)thio) benzamide (compound 80)

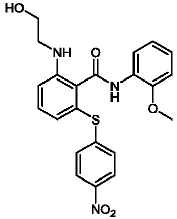

Prepared by proceeding in similar manner to example 9, Yellow solid; 66% yield.
¹H NMR (400 MHz, CDCl₃) *δ* = 3.05 (t, *J* = 8.0Hz, 2H), 3.66 (m, 2H), 3.90 (s, 3H), 6.44 (d, *J* = 8.0Hz, 1H), 6.78 (d, *J* = 8.0Hz, 1H), 7.02 (m, 3H), 7.37 (m, 4H), 7.57 (m, 1H), 7.97 (d, *J* = 7.2Hz, 2H), 8.19 (s, 1H).
ESI-MS: [M+ Na]⁺ m/z 460

### Comparative Example 34

### General Procedure for 2-((2-hydroxyethyl)amino)-N-(2-methoxyphenyl)-6-((4-nitrophenyl)sulfonyl) benzamide (compound 81)

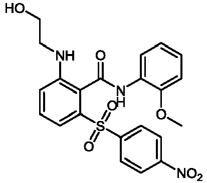

Prepared by proceeding in similar manner to example 7, yellow solid; 43% yield;
¹H NMR (400 MHz, CDCl₃) *δ* = 3.68 (m, 2H), 3.96 (t, *J* = 8.4Hz, 2H), 4.02 (s, 3H), 6.69 (d, *J* = 8.0Hz, 1H), 7.07 (t, *J* = 8.0Hz, 1H), 7.14 (d, *J* = 8.0Hz, 1H), 7.24 (m, 3H), 7.43 (m, 2H), 7.57 (d, *J* = 8.0Hz, 3H), 7.99 (d, *J* = 7.6Hz, 2H);
ESI-MS: [M+ Na]⁺ m/z 494.

### Comparative Example 35

### General Procedure for 2-((2-amino-2-oxoethyl)amino)-N-(2-methoxyphenyl)-6-((4-nitrophenyl)thio) benzamide(compound 82)

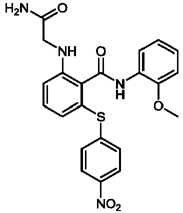

Prepared by proceeding in similar manner to example 9, White solid; 86.1% yield;
¹H NMR (400 MHz, DMSO) *δ* = 3.78 (s, 3H), 5.34 (brs, 2H), 5.85 (s, 2H), 6.46 (m, 2H), 6.67 (t, *J* = 7.2Hz, 2H), 6.76 (t, *J* = 7.2Hz, 2H), 6.99 (d, *J* = 8.0Hz, 2H), 7.16 (m, 3H), 7.43 (d, *J* = 7.2Hz, 1H), 7.55 (s, 1H).
ESI-MS: [M+ H]⁺ m/z 453.

### Example 9

### General Procedure for 2-(2-amino-3-phenylpropanamido)-N-(2-methoxyphenyl)-6-((4-nitrophenyl) sulfonyl)benzamide (compound 83)

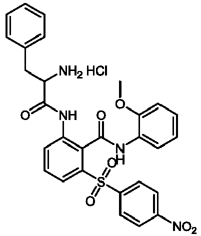

Prepared by proceeding in similar manner to example 31, Yellow solid; 43% yield;
¹H NMR (400 MHz, DMSO) *δ* = 2.88 (m, 1H), 3.14 (d, *J* = 16.0Hz, 2H), 3.44 (d, *J* = 8.0Hz, 1H), 3.69 (s, 3H), 4.35 (brs, 2H), 6.99 (m, 2H), 7.12 (t, *J* = 8.0Hz, 1H), 7.23 (d, *J* = 8.0Hz, 4H), 7.77 (t, *J* = 8.0Hz, 1H), 7.94 (d, *J* = 4.0Hz, 1H), 8.10 (d, *J* = 8.0Hz, 1H), 8.31 (m, 7H), 9.88 (s, 1H).
ESI-MS: [M+ H]⁺ m/z 575.

### biological activity tests

The anti-HIV activity was determined by the "Guiding Principles of Anti HIV Drug Unclinical Pharmacodynamics" of SFDA which is tested by technology standard assay according to International Universal Standards and Methods. The results are shown in Table 1. Where in CC₅₀, EC₅₀ were measured twice and took the average.

From Table 1, we can see that most of the compounds can inhibit HIV in vitro. Especially compound 25, 46, 67 and 83, which bearing high efficiency, low toxicity and high TI. Thus these compounds are suitable to be novel inhibitors of vif for anti-HIV.

**Table 1**

| **compound** | **C8166 cells** | | | **H9 cells** | | |
|---|---|---|---|---|---|---|
| | CC₅₀ | EC₅₀ | TI | CC₅₀ | EC₅₀ | TI |
| **22** | 107.83 | >200 | 0.54 | >200 | >200 | - |
| **23** | >200 | >200 | - | >200 | >200 | - |
| **24** | 172.21 | 2.59 | 66.58 | >200 | 2.62 | >76.34 |
| **25** | 65.40 | 0.21 | 310.02 | 152.94 | 0.74 | 206.68 |
| **27** | 80.57 | 24.03 | 3.35 | 123.48 | 98.45 | 1.25 |
| **28** | 93.04 | 83.84 | 1.11 | 77.23 | 98.45 | 0.78 |
| **29** | 109.72 | 73.50 | 1.49 | 74.62 | >200 | <0.37 |
| **45** | >200 | 0.367 | 385.34 | 117.08 | 1.66 | 70.53 |
| **46** | 140.59 | 0.096 | 2081.59 | >200 | 0.66 | >303.03 |
| **48** | - | - | - | 76.60 | 35.87 | 2.14 |
| **49** | - | - | - | >200 | 41.50 | 4.82 |
| **50** | - | - | - | 81.06 | 28.29 | 2.87 |
| **51** | - | - | - | 18.09 | 18.84 | 0.96 |
| **52** | - | - | - | >200 | >200 | - |
| **53** | - | - | - | >200 | 43.69 | >4.58 |
| **54** | - | - | - | 85.79 | 83.93 | 1.02 |
| **55** | >200 | >200 | - | >200 | >200 | - |
| **56** | >200 | 24.48 | >8.17 | >200 | 137.08 | >1.46 |
| **57** | 37.24 | 14.07 | 2.65 | >200 | 16.81 | >11.90 |
| **58** | >200 | 99.97 | >2.00 | >200 | 102.89 | >1.94 |
| **59** | >200 | 63.68 | >3.14 | 88.39 | 122.90 | 0.72 |
| **60** | >200 | 91.32 | >2.19 | >200 | >200 | - |
| **61** | >200 | 46.94 | >4.26 | >200 | >200 | - |
| **62** | 10.25 | 3.43 | 2.99 | 14.22 | 3.83 | 3.71 |
| **63** | >200 | 53.31 | >3.75 | >200 | >200 | - |
| **64** | 10.424 | 3.17 | 3.28 | 17.83 | 21.01 | 0.85 |
| **65** | 18.789 | 105.78 | 0.18 | 25.41 | >200 | 0.13 |
| **66** | 78.263 | 0.81 | 97.01 | 72.27 | 0.16 | 451.69 |
| **67** | 165.488 | 0.43 | 388.04 | 94.87 | 0.12 | 790.58 |
| **68** | 115.617 | 0.94 | 122.45 | 90.09 | 2.38 | 37.85 |
| **71** | >200 | 61.44 | >3.26 | >200 | >200 | - |
| **72** | >200 | 16.19 | >12.35 | >200 | 192.60 | 1.04 |
| **73** | 194.07 | 36.73 | 5.28 | >200 | >200 | - |
| **74** | 68.60 | 7.26 | 9.45 | >200 | >200 | - |
| **75** | >200 | >200 | - | >200 | >200 | - |
| **76** | >200 | 92.05 | >2.17 | >200 | 100.3 | 1.99 |
| **77** | >200 | >200 | - | >200 | >200 | - |
| **78** | 24.23 | 16.94 | 1.43 | 16.75 | 19.91 | 0.84 |
| **79** | 90.86 | 93.53 | 0.97 | 99.47 | 100.82 | 0.99 |
| **80** | 93.51 | 19.44 | 4.81 | 116.38 | 19.15 | 6.08 |
| **81** | 102.26 | 89.10 | 1.15 | 72.70 | 101.99 | 0.71 |
| **82** | >200 | >200 | - | >200 | >200 | - |
| **83** | 178.23 | 0.45 | 396.07 | 102.44 | 0.15 | 682.93 |

| | | | | | | |
|---|---|---|---|---|---|---|
| CC₅₀, EC₅₀ (µg/mL) | | | | | | |

Compound 67 and 83 are prodrugs of compound 46 (In C8166 TI= 2081.59, in H9 TI=303.03). It was proved that they were hydrolyzed into 46 in vivo. It should be noted that the prodrugs also have the anti-HIV activity of their own (In C8166 TI= 388.04 and 291.4, in H9 TI=790.58 and 756.32).

**Anti-HIV-1 activities of 67 and 46**

| | C8166 | | | H9 | | |
|---|---|---|---|---|---|---|
| **compound** | CC₅₀ (µg/mL) | EC₅₀ (µg/mL) | TI | CC₅₀ (µg/mL) | EC₅₀ (µg/mL) | TI |
| **67** | 165.488 | 0.43 | 388.04 | 94.87 | 0.12 | 790.58 |
| **46** | 140.59 | 0.096 | 2018.59 | >200 | 0.66 | >303.03 |

The anti-HIV activities of 46 against different resistant viruses were also tested and showing low toxicity toward mutant strain. 46 can inhibit the pathological changes of C8166 induced by HIV-1_{IIIB} as well as the reproduction of viruses in H9 cells. 46 has a good inhibition on HIV-1_{74V}, HIV-1_{A17}, and HIV-1_{L10R/M46I/L63P/V82T/I84V} as well as Clinical isolated strain such as HIV-1_{KM018} and HIV-1_{TC-1}. (Table 2).

**Table 2: Anti-HIV-1 activities of 46 in cell cultures^{a}**

| **Virus** | EC₅₀(µg/mL) | TI(CC₅₀/EC₅₀) |
|---|---|---|
| **HIV-1_{IIIB} (C8166 cells)** | 0.096 | 2081.59 |
| **HIV-1_{IIIB}(H9 cells)** | 0.66 | >303.03 |
| **HIV-1_{74V}** | 0.81 | 173.57 |
| **HIV-1_{A17}** | 18.72 | >20 |
| **HIV-1_{L10R/M46I/L63P/V82T/I84V}** | 0.83 | 169.39 |
| **HIV_{KM018}** | 6.23 | >31.65 |
| **HIV-1_{TC-1}** | 0.11 | >1818.18 |

| | | |
|---|---|---|
| ^{a}C8166 cells and H9 cells: human T-lymphoma cell line; HIV-1_{IIIB} and HIV-1_{74V}: NNRTI-resistant virus strains; HIV-1_{A17}: NVP resistant virus strains; HIV-1_{L**10R/M46I/L63P/V82T/I84V**}: PI-resistant virus strains; HIV_{KM018} and HIV-1_{TC-1}: clinical strains. | | |

Meanwhile these compounds have good therapeutic effect in HIV-2. Compound 25, 46, 67 and 83 bear high TI and low toxicity.

**Table 3: the active of compound 25, 46, 67 for HIV-2 ROD and HIV-2CBL-20**

| | **HIV-2 ROD** | | | **HIV-2 CBL-20** | | |
|---|---|---|---|---|---|---|
| **compound** | CC₅₀ (µg/ml) | EC50 (µg/ml) | TI | CC₅₀ (µg/ml) | EC50 (µg/ml) | TI |
| **25** | 184.61 | 0.59 | 312.90 | 163.77 | 0.82 | 199.72 |
| **46** | >200 | 0.41 | 487.8 | >200 | 0.45 | 444.44 |
| **67** | 126.19 | 0.78 | 161.78 | 106.26 | 1.21 | 87.82 |

| | | | | | | |
|---|---|---|---|---|---|---|
| HIV-2 ROD Separated and got at Senegal in 1985; HIV-2CBL-20 Separated and got at Gambia. | | | | | | |

Solubility is an important parameter of druggability. The solubility of prodrugs 66, 67 and 68 are good. The solubility of of 67 in water with pH=7 can be as good as 1730.64µg/ml. The solubility of 66 and 68 in aqueous solution (pH=2) can reach 1290 and 2845.5µg/ml. they are with good solubility and can be formulated into an oral formulation. The prodrugs can be hydrolyzed into 46 which is with good fat-solubility and TI as well as distribution.

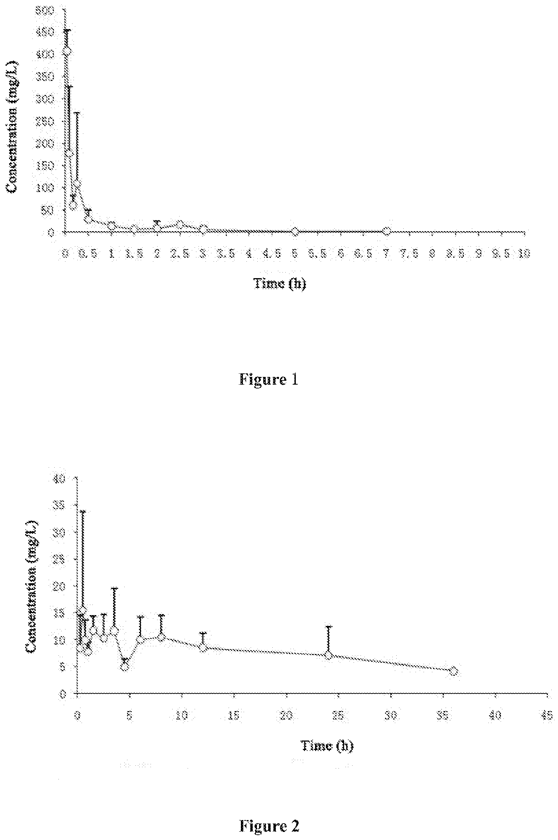

In pharmacokinetics, after oral gavage of 67 by 100mg/kg, the max blood concentration of 46 can be as high as 21.662µg/ml and it can be relased in many times in vivo, reaching a bioavailability of 161.2%. (fig.1 and fig.2)

**The metabolism of 67 to 46 medicine curve and bioavailability**

| | **Cmax(mg/L)** | **AUC(0∼)9(mg/L*h)** | **CL(L/h/kg)** | **F(%)** |
|---|---|---|---|---|
| **oral (100mg/kg)** | 21.662 | 387.758 | 4.878 | 162.2 |
| **Intravenous(50mg/kg)** | 326.494 | 120.282 | 0.453 | |

In toxicity test, the acute toxicity test of 67 and 46 were tested and no abnormal phenomenon occurred.

In pharmacodynamics, results show that 46 and its prodrug 67 are very safe even at a high dose (10g/kg).

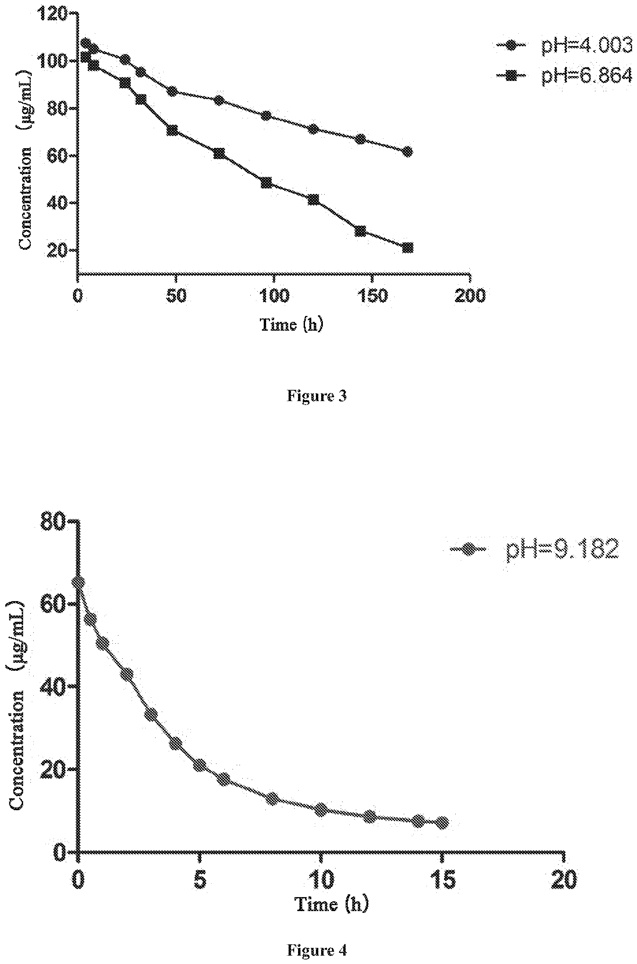

As for the stability of the drugs, those prodrugs possess the favorable stability by experimentation. For example, compound 67 was tested in solutions with PH = 4.004, 6.864 and 9.182, and its degradation rate was 0.2767, 0.4956 and 9.182, respectively. 67 possess the favorable stability in acid condiction while they prone to be hydrolyzed in alkaline solution. As to the Mechanism of action of the compounds, the possible binding mode between 46 and vif was predicted by docking. There are five possible hydrogen bonds between them: two hydrogen bonds between the nitro group and Arg79 and Lys178, two hydrogen bonds between the oxygen atom of methoxyl group with His141 and Arg97, one hydrogen bond between the nitrogen atom of pridine with Gly103. (The binding mode can be seen in figure 4)

## Claims

1. An anti-HIV compound having the following formula (III-11-111): 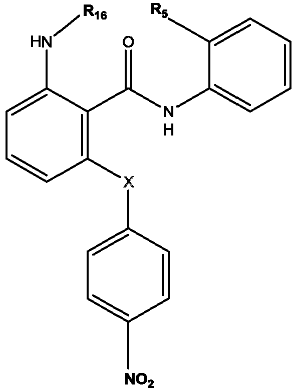 Wherein:
R₅ is a straight or branched C₁₋₈ alkoxy group;
X is S, O or SO_{2;}
R₁₆ is H;
or an anti-HIV compound as represented by a structure selected from the following group: 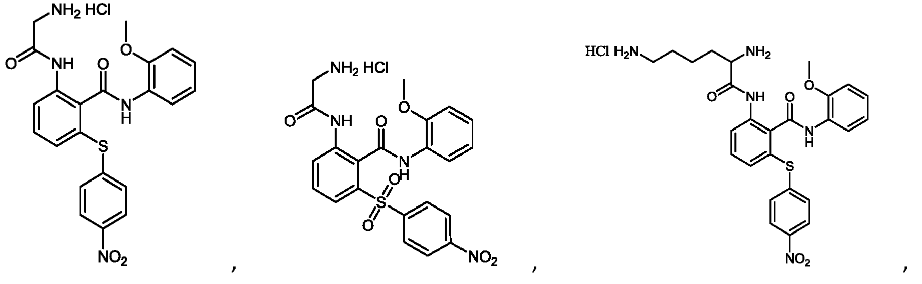 or

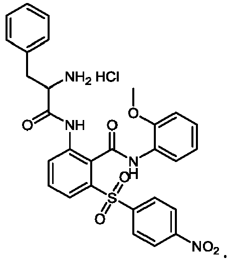

2. The compound of formula III-11-111 as claimed in claim 1 **characterized in that** R₅ is C₁₋₄ alkoxy group.

3. The compound of formula III-11-111 as claimed in claim 1 **characterized in that** R₅ is C₁₋₂ alkoxy group.

4. The compound of formula III-11-111 as claimed in claim 1 **characterized in that** R₅ is methoxyl.

5. An anti-HIV compound as claimed in any preceding claim represented by a structure selected from the following group:

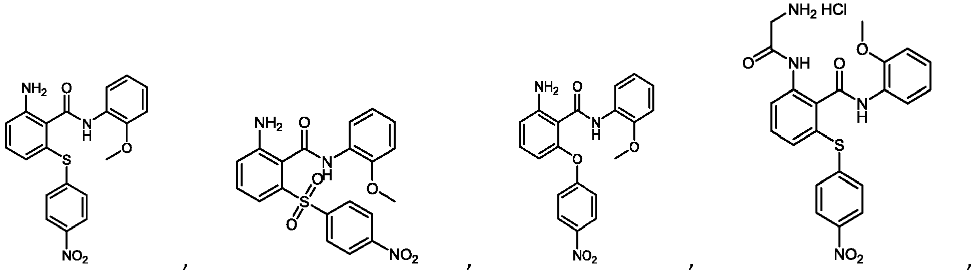

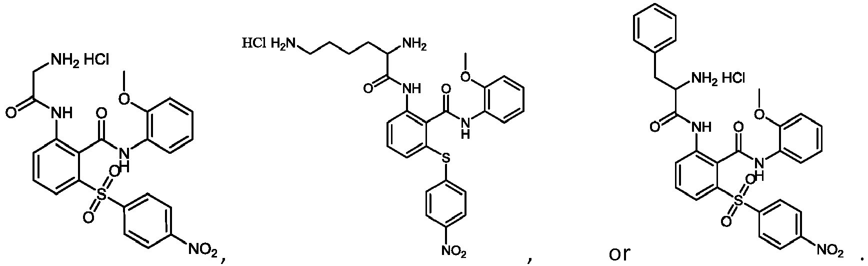

6. A pharmaceutically acceptable salt of said compound as claimed in any preceding claim wherein the said salt refers to hydrochloride, sulfate, phosphate or nitrate.

7. The pharmaceutically acceptable salt as claimed in claim 6 wherein the said salt refers to hydrochloride.

8. Use of a compound according to any one of the claims 1 to 5 for the manufacture of a medicament for treating HIV, neoplastic diseases or hepatitis.

9. Use of a compound according to any one of the claims 1 to 5 for the manufacture of a medicament for treating HIV, wherein the HIV disease is HIV-1 or HIV-2.

## Patentansprüche

1. Eine Anti-HIV-Verbindung der folgenden Formel (III-11 -111): 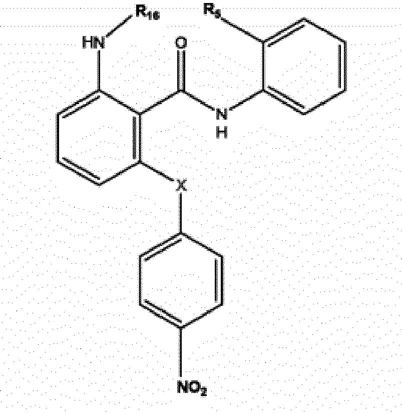 worin:
R₅ eine gerade oder verzweigte C₁₋₈-Alkoxygruppe ist;
X ist S, O oder SO₂;
R₁₆ ist H;
oder eine Anti-HIV-Verbindung, die durch eine Struktur dargestellt ist, die aus der folgenden Gruppe ausgewählt wird:

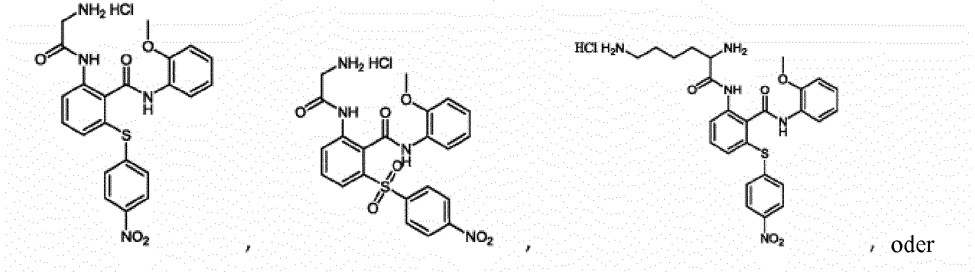

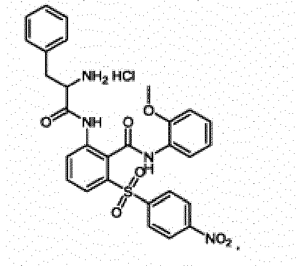

2. Die Verbindung der Formel III-11-111 nach Anspruch 1, **dadurch gekennzeichnet, dass** R₅ eine C₁₋₄ -Alkoxygruppe ist.

3. Die Verbindung der Formel III-11-111 nach Anspruch 1, **dadurch gekennzeichnet, dass** R₅ eine C₁₋₂ -Alkoxygruppe ist.

4. Die Verbindung der Formel III-11-111 nach Anspruch 1, **dadurch gekennzeichnet, dass** R₅ Methoxygruppe ist.

5. Eine Anti-HIV-Verbindung nach einem der vorhergehenden Ansprüche, die durch eine Struktur dargestellt ist, die aus der folgenden Gruppe ausgewählt wird:

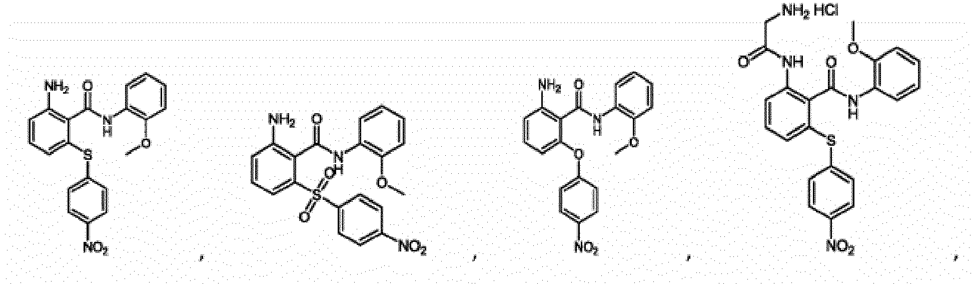

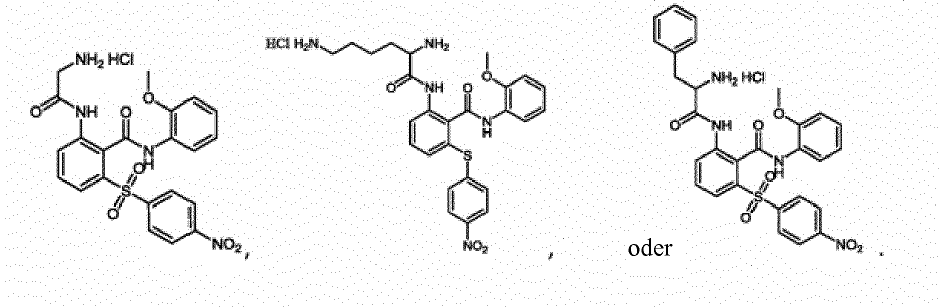

6. Ein pharmazeutisch verträgliches Salz der Verbindung nach einem der vorhergehenden Ansprüche, wobei sich das beschriebene Salz auf Hydrochlorid, Sulfat, Phosphat oder Nitrat bezieht.

7. Das pharmazeutisch verträgliche Salz nach Anspruch 6, wobei sich das beschriebene Salz auf Hydrochlorid bezieht.

8. Verwendung einer Verbindung nach einem der Ansprüche 1 bis 5 zur Herstellung eines Medikaments zur Behandlung von HIV, neoplastischen Erkrankungen oder Hepatitis.

9. Verwendung einer Verbindung nach einem der Ansprüche 1 bis 5 zur Herstellung eines Medikaments zur Behandlung von HIV, wobei die HIV-Erkrankung HIV-I oder HIV-2 ist.

## Revendications

1. Composé anti-VIH possédant la formule suivante (III-11-111): 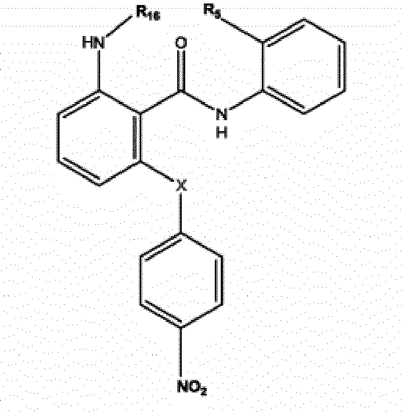 Où:
R₅ est un groupe alcoxy en C₁₋₈ à chaîne droite ou ramifié;
X est S, O ou SO₂;
R₁₆ est H;
ou un composé anti-VIH tel que représenté par une structure choisie dans le groupe suivant:

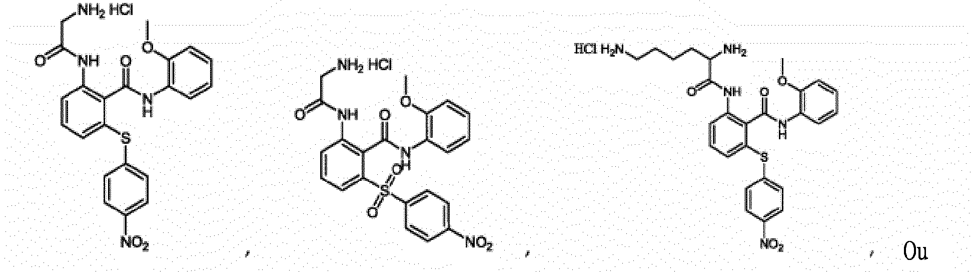

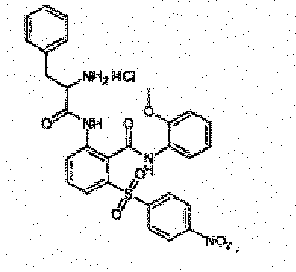

2. Composé de formule III-11-111 selon la revendication 1, **caractérisé en ce que** R₅ est un groupe alcoxy C₁₋₄.

3. Composé de formule III-11-111 selon la revendication 1, **caractérisé en ce que** R₅ est un groupe alcoxy C₁₋₂.

4. Composé de formule III-11-111 selon la revendication 1, **caractérisé en ce que** R₅ est un mé thoxyle.

5. Composé anti-VIH selon l'une quelconque des revendications précédentes, représenté par une structure choisie dans le groupe suivant:

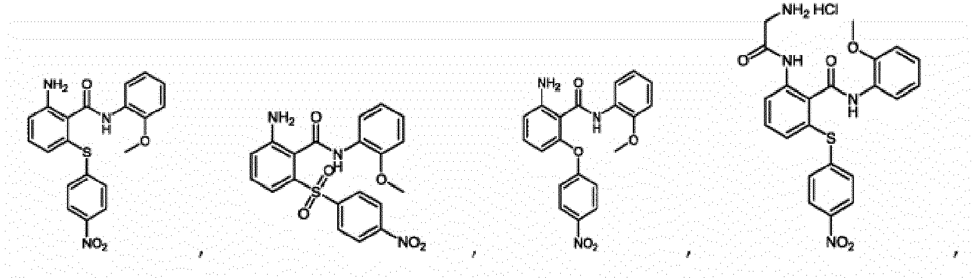

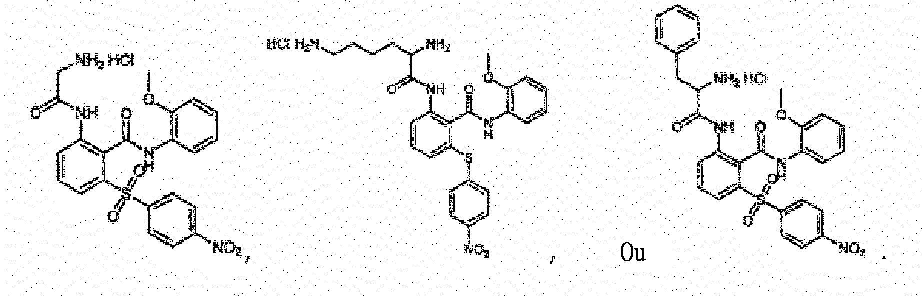

6. Sel pharmaceutiquement acceptable dudit composé selon l'une quelconque des revendications précédentes, dans lequel ledit sel désigne un chlorhydrate, un sulfate, un phosphate ou un nitrate.

7. Sel pharmaceutiquement acceptable selon la revendication 6, dans lequel ledit sel se réfère à un chlorhydrate.

8. Utilisation d'un composé selon l'une quelconque des revendications 1 à 5 pour la fabrication d'un médicament destiné au traitement du VIH, des maladies néoplasiques ou de l'hépatite.

9. Utilisation d'un composé selon l'une quelconque des revendications 1 à 5 pour la fabrication d'un médicament pour le traitement du VIH, dans laquelle la maladie à VIH est le VIH-I ou le VIH-2.
